# EUROPEAN PATENT APPLICATION

(11) **EP 1 447 092 A1**
(43) Date of publication of application: **18.08.2004**
(21) Application number: 02800255.8
(22) Date of filing: 27.09.2002
(51) Int. Cl.: A61K 39/00, A61K 45/00, A61P 35/00

(54) **METHODS OF INDUCING ANTIGEN-SPECIFIC T CELLS**

(30) Priority: 28.09.2001 JP 2001301224
(71) Applicant: Sugiyama, Haruo, Minoo-shi, Osaka 562-0036 (JP); Azuma, Ichiro, Sapporo-shi Hokkaido 005-0012 (JP)
(72) Inventor: Sugiyama, Haruo, Minoo-shi, Osaka 562-0036 (JP); Azuma, Ichiro, Sapporo-shi Hokkaido 005-0012 (JP)
(74) Representative: VOSSIUS & PARTNER
(86) International application number: PCT/JP2002/009997
(87) International publication number: WO 2003/028758

(57) **Abstract**

The present invention provides a novel method for inducing antigen-specific T cells. A method for inducing antigen-specific T cells in a patient comprising administering to said patient in need thereof composition (a) which comprises a therapeutically effective amount of an antigen protein or an antigen peptide as an active ingredient, and composition (b) which comprises a therapeutically effective amount of a cell wall skeleton integrant of the BCG strain of *Mycobacterium bovis* as an active ingredient, wherein composition (b) is administered in advance and then composition (a) is administered, and related pharmaceutical compositions are provided.

## Description

### FILED OF THE INVENTION

The present invention relates to novel methods of inducing antigen-specific T cells. In particular, the present invention relates to methods of inducing antigen-specific T cells, which administering a composition comprising a cell wall skeleton integrant of the BCG strain of *Mycobacterium bovis* (hereinafter, as referred to as BCG-CWS) in advance and then administering a composition comprising an antigen protein or an antigen peptide. In addition, the present invention relates to compositions for treating and/or preventing cancers that are characterized in that they comprise a cancer antigen protein, WT1, or a cancer antigen peptide derived from said WT1 protein in combination with a BCG-CWS.

### BACKGROUND ART

Cellular immunity mediated by, among others, cytotoxic T cells (also sometimes referred to as killer T cells or CTLs) or helper T cells, which are antigen-specific T cells, plays a central role in elimination of cancer cells or virus-infected cells from a living body. An antigen-specific T cell recognizes, using its T cell receptor, a bound complex between an MHC molecule (also referred to as an HLA molecule in case of human) on the cell surface of an antigen-presenting cell such as a dendritic cell or macrophage and an antigen peptide which is a fragment peptide of an antigen protein derived from a cancer or virus, and thereby differentiates and proliferates. Antigen peptides presented on the MHC molecules are known to be usually about 8 to 20 amino acids in length. Antigen-specific T cells that thus have differentiated and proliferated exert their anti-tumor or anti-viral effects by specifically injuring cancerous or virus-infected cells that present the complex bound between the antigen peptide and the MHC molecule, or by producing various cytokines.

So-called vaccine therapies in which an antigen protein or an antigen peptide derived from a cancer or virus is administered to potentiate antigen-specific T cells are believed useful for treatment or prevention of cancers and viral infections. Cancerous or viral antigens recognized by T cells have been screened to date for various cancers and virus infections, and many cancer antigen proteins, virus-derived antigen proteins, and antigen peptides derived therefrom have been already identified (Immunogenetics 1995, 41:178; Cancer Immunol. Immunother. 2001, 50:3). For example, one of those antigens, WT1, was originally identified as a causative gene for a childhood renal tumor, that is, Wilms tumor (Nature 1990, 343:774). In normal tissues, the WT1 gene is weakly expressed only in restricted tissues such as kidney, testis, and ovary, whereas it has been shown to be highly expressed in various cancers such as leukemia as well as lung, breast, ovarian, prostatic, bladder, uterine, cervical, gastric, colon, germ cell, hepatic, and skin cancers (JP Kokai H09-104627, JP Kokai H11-35484). Recently, it has been shown that WT1-specific cytotoxic T cells (CTLs) were induced by *in vitro* stimulation of peripheral blood niononuclear cells from HLA-A2.1- or HLA-A24.2-positive human donors with a 9-mer WT1 peptide comprising an MHC class I binding motif (Immunogenetics 51:99-107, 2000; Blood 95:2198-203, 2000; Blood 95:286-93, 2000). It has also been shown that WT1-specific CTLs were induced by *in vivo* immunization of mice with a 9-mer WT1 peptide (J Immunol 164:1873-80, 2000; Blood 96:1480-9, 2000) or WT1 cDNA (J Clin Immunol 20:195-202, 2000), and further that the immunized mice reject transplanted tumor cells highly expressing WT1 (J Immunol 164: 187.3-80, 2000; J Clin Immunol 20:195-202, 2000). These findings demonstrate that WT1 protein is one of cancer antigen proteins, and may provide a measure for cancer vaccines against fluid or solid cancers.

In order to efficiently induce a specific immunity by vaccination, it is effective to administer an antigen protein or an antigen peptide as a principal agent in combination with a non-specific immunopotentiator. Known non-specific immunopoterltiators include bacterium-derived components, cytokines, plant-derived components, and marine organism-derived components. Bacterium-derived components include a dead body of the BCG strain of *Mycobacterium bovis,* a cell wall skeleton integrant of said BCG strain (BCG-CWS), a human tubercle bacillus-derived polysaccharide material (e.g. Ancer), a hemolytic streptococcus powders (e.g. Picibanil), a bacterium-derived polysaccharide (e.g. lentinan, Krestin), a dead microbial suspension cocktail (e.g. Broncasma Berna), a muramyldipeptide (MDP)-related compound, a lipopolysaccharide (LPS), a lipid A-related compound (MPL), a glycolipid trehalose dimycolate (TDM), and a DNA derived from the bacteria as mentioned above (e.g. CpG oligonucleotides). Among them, a BCG-CWS has been known to exhibit an effective immunopotentiating action when dispersed in an oil such as mineral oils (Cancer Res., 33, 2187-2195 (1973); J. Nat. Cancer Inst., 48, 831-835 (1972); J. Bacteriol., 94, 1736-1745 (1967); Gann, 69, 619-626 (1978); J. Bacteriol., 92, 869-879 (1966)).

In addition, the dosage form of vaccine is also an important factor for efficient induction of specific immunity. For example, aluminium preparations, lipid particles, emulsion preparations, and microspheres are known as dosage forms of vaccines.

These substances and dosage forms that effect the enhancement of vaccine efficacies are collectively called adjuvants (Nature Biotech. 1999, 17:1075). At present, the most widely used adjuvant among those as approved for human use is an aluminium preparation, but its ability to induce antigen-specific T cells is low, and side effects such as IgE production have been pointed out as problems.

In the light of the great ability of dendritic cells as antigen-presenting cells to induce antigen-specific T cells, research on cell vaccines has been also conducted in recent years, in which dendritic cells derived from a patient are pulsed *in vitro* with an antigen protein or an antigen peptide to cause antigen presentation, and then put back into the patient (Nature Med. 1998, 4:328). However, there are many problems to be solved before the cell vaccine therapy is widely available; it is technically difficult and costly to obtain a large amount of dendritic cells required for the therapy.

Under such circumstances, there has been a need for developing a novel vaccine that enables simple, convenient, and efficient induction of antigen-specific T cells as well as a method of administering the same.

### DISCLOSURE OF THE INVENTION

An object of the present invention is to provide a novel method by which antigen-specific T cells can be efficiently induced. The object is thus to provide a method of inducing antigen-specific T cells which comprises administering a composition comprising a non-specific immunopotentiator, BCG-CWS, in advance, and then administering a composition comprising an antigen protein or an antigen peptide. A further object of the present invention is to provide a composition for treating and/or preventing cancers that is characterized in that it comprises a cancer antigen protein, WT1, or a cancer antigen peptide derived from said WT1 protein in combination with a BCG-CWS.

In order to elicit immune responses to a vaccine, it is important to timely administer an antigen in combination with an appropriate non-specific immunopotentiator. As described above, however, there has not been known any method of administering a vaccine that efficiently induces antigen-specific T cells and thereby produces anti-tumor or anti-viral effects.

The present inventors therefore concentrated their efforts on examining therapeutic effects on an *in vivo* cancer model, of the use of cancer antigen peptides derived from the cancer antigen protein WT1, as used as an example, in combination with a non-specific immunopotentiator, BCG-CWS. As a result, it was found for the first time that a drastic improvement in the induction of antigen-specific T cells, leading for example to anti-tumor effects, can be obtained by a novel approach wherein a BCG-CWS is administered in advance and, after a certain period, an antigen peptide is administered, compared to the approaches wherein an antigen or a BCG-CWS is solely administered. The inventors believe that this administration method should provide a similar effect whatever antigen protein or antigen peptide is combined with a BCG-CWS.

Furthermore, in the present invention, the cancer antigen protein WT1 was, for the first time, examined for its effects in a cancer model that reflects, so to speak, "a therapeutic system" in which a cancer antigen peptide derived from WT1 is administered after the transplantation of tumor cells into an animal. As a result, it was demonstrated for the first time that WT1 is therapeutically effective. In addition, it was found that cancer antigen peptides derived from said WT1 protein produce remarkable anti-tumor effects when administered in combination with a BCG-CWS.

The present invention is based on such findings as described above.

Thus, the present invention relates to:
(1) a method for inducing antigen-specific T cells in a patient comprising administering to said patient in need thereof;
   composition (a) which comprises a therapeutically effective amount of an antigen protein or an antigen peptide as an active ingredient, and
   composition (b) which comprises a therapeutically effective amount of a cell wall skeleton integrant of the BCG strain of *Mycobacterium bovis* (BCG-CWS) as an active ingredient, wherein composition (b) is administered in advance and then composition (a) is administered;
(2) A method according to above (1), wherein composition (a) is administered about 24 hours after the administration of composition (b);
(3) A method according to above (1) or (2), wherein compositions (a) and (b) are both administered intradermally;
(4) A method according to above (3), wherein compositions (a) and (b) are both administered intradermally at the same site;
(5) A method according to any one of above (1) to (4), wherein the administration cycle involving compositions (a) and (b) is repeated two or more times;
(6) A method according to any one of above (1) to (5); wherein composition (a) comprises a cancer antigen protein or a cancer antigen peptide as an active ingredient;
(7) A method according to above (6), wherein the cancer antigen protein or the cancer antigen peptide is WT1 protein set forth in SEQ ID NO: 1 or a cancer antigen peptide derived from said WT1 protein;
(8) A method according to above (7), wherein the cancer antigen peptide derived from WT1 protein is, selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4);
(9) A method of treatment and/or prevention of a cancer in a patient which comprises a method according to any one of above (1) to (8); and
   in another embodiment,
(10) A pharmaceutical composition for enhancing an activity of an antigen protein or an antigen peptide to induce antigen-specific T cells, which comprises a cell wall skeleton integrant of the BCG strain of *Mycobacterium bovis* as an active ingredient, and which is administered before the administration of the antigen protein or the antigen peptide;
(11) A pharmaceutical composition for enhancing an anticancer activity based on the immunopotentiating action of a cell wall skeleton integrant of the BCG strain of *Mycobacterium bovis,* which comprises an antigen protein or an antigen peptide as an active ingredient, of which the activity to induce antigen-specific T cells facilitates the enhancement of the anticancer activity, said composition being administered after the administration of the cell wall skeleton integrant of the BCG strain of *Mycobacterium bovis;*
(12) A pharmaceutical composition according to above (10) or (11), wherein the antigen protein or the antigen peptide is a cancer antigen protein or a cancer antigen peptide;
(13) A pharmaceutical composition according to above (12), wherein the cancer antigen protein or the cancer antigen peptide is WT1 protein set forth in SEQ ID NO: 1 or a cancer antigen peptide derived from said WT1 protein;
(14) A pharmaceutical composition according to claim 13, wherein the cancer antigen peptide derived from WT1 is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4);
(15) A pharmaceutical composition according to any one of above (10) to (14) for treatment and/or prevention of a cancer; and
   in relation to the embodiment as mentioned above,
(16) A use of a cell wall skeleton integrant of the BCG strain of *Mycobacterium bovis* for preparing a medicament which is administered before the administration of an antigen protein or an antigen peptide and which enhances an activity of the antigen protein or the antigen peptide to induce antigen-specific T cells;
(17) A use of an antigen protein or an antigen peptide for preparing a medicament which is administered after the administration of a cell wall skeleton integrant of the BCG strain of *Mycobacterium bovis* and which enhances an anticancer activity based on the immunopotentiating action of said a cell wall skeleton integrant of the BCG strain of *Mycobacterium bovis,* wherein the anticancer activity is enhanced by the activity of the cancer antigen protein or the cancer antigen peptide to induce antigen-specific T cells; and a use corresponding to the embodiments according to above (12) to (15); and
   in another embodiment,
(18) A pharmaceutical composition for enhancing an activity of WT1 protein set forth in SEQ ID NO: 1 or a cancer antigen peptide derived from said WT1 protein to induce antigen-specific T cells, which comprises a cell wall skeleton integrant of the BCG strain of *Mycobacterium bovis* (BCG-CWS) as an active ingredient;
(19) A pharmaceutical composition for enhancing an anticancer activity based on the immunopotentiating action of a cell wall skeleton integrant of the BCG strain of *Mycobacterium bovis* (BCG-CWS), which comprises as an active ingredient WT1 protein set forth in SEQ ID NO: 1 or a cancer antigen peptide derived from said WT1 protein, of which the activity to induce antigen-specific T cells facilitates the enhancement of the anticancer activity;
(20) A pharmaceutical composition according to above (18) or (19), wherein the cancer antigen peptide derived from WT1 is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4);
(21) A pharmaceutical composition according to any one of above (18) to (20) for treatment and/or prevention of a cancer; and
   in relation to the embodiment as mentioned above,
(22) A method for enhancing, in a patient, an activity of WT1 protein set forth in SEQ ID NO: 1 or a cancer antigen peptide derived from said WT1 protein to induce antigen-specific T cells, which comprises administering to the patient a cell wall skeleton integrant of the BCG strain of *Mycobacterium bovis* in an amount effective to enhance the activity to induce the antigen-specific T cells;
(23) A method for enhancing, in a patient, an anticancer activity based on the immunopotentiating action of a cell wall skeleton integrant of the BCG strain of *Mycobacterium bovis,* which comprises administering to the patient WT 1 protein set forth in SEQ ID NO: 1 or a cancer antigen peptide derived from said WT1 protein in an amount effective to enhance the anticancer activity, wherein the anticancer activity is enhanced by the activity of the cancer antigen protein or the cancer antigen peptide to induce antigen-specific T cells;
(24) A method according to above (22) or (23), wherein the cancer antigen peptide derived from WT1 protein is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4);
(25) A method according to any one of above (22) to (24) for treatment and/or prevention of a cancer;
(26) A use of a cell wall skeleton integrant of the BCG strain of *Mycobacterium bovis* for preparing a medicament which enhances an activity of WT1 protein set forth in SEQ ID NO: 1 or a cancer antigen peptide derived from said WT1 protein to induce antigen-specific T cells;
(27) A use of WT1 protein set forth in SEQ ID NO: 1 or a cancer antigen peptide derived from WT1 protein for preparing a medicament which enhances an anticancer activity based on the immunopotentiating action of a cell wall skeleton integrant of the BCG strain of *Mycobacterium bovis,* wherein the anticancer activity is enhanced by the activity of the cancer antigen protein or the cancer antigen peptide to induce antigen-specific T cells;
(28) A use according to above (26) or (27), wherein the cancer antigen peptide derived from WT1 protein is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4); and
(29) A use according to any one of above (26) to (28) for preparing a medicament for treatment and/or prevention of a cancer.

### BRIEF DESCRIPTION OF DRAWINGS

Fig. 1 shows a schedule for transplantation of tumor cells and vaccination with a WT1 peptide and a BCG-CWS.
Fig. 2 is a graph showing the longer diameters of tumors in mm up until Day 65 after transplantation of tumor cells on vaccinations.
   In the figure, solid circles indicate results of WT1 peptide + BCG-CWS administration, open triangles indicate results of WT1 peptide administration, solid diamonds indicate results of BCG-CWS administration, and solid squares indicate results without any vaccination.
Fig. 3 is a graph showing the survival ratio (%) of mice up until Day 65 after transplantation of tumor cells on vaccinations. In the figure, - indicates the results WT1 peptide + BCG-CWS administration, ―•―• indicates the result of WT 1 peptide administration, ----- indicates the result of BCG-CWS administration, and •••• indicates the result without any vaccination.
Fig. 4 is a graph showing the disease free survival rate (%) of mice up until Day 65 after transplantation of tumor cells on vaccinations. In the figure, ― indicates the results of WT1 peptide + BCG-CWS administration, ―•―• indicates the result of WT 1 peptide administration, ----- indicates the result of BCG-CWS administration, and •••• indicates the result without any vaccination.
Fig. 5 is a graph showing the results of colony assay of the myeloid cells removed on Day 65 from the mice transplanted with tumor cells and vaccinated. In the figure, solid bars indicate results of WT1 peptide + BCG-CWS administration, stippled bars indicate results of BCG-CWS administration, and open bars indicate results without any vaccination.
Fig. 6 is a graph showing the cytotoxic effects of splenocytes from the mice that any tumor has not been established by coadministration of WT1 peptide and BCG-CWS and from the untreated mice on WT1-C1498 cells and C 1498 cells.

### BEST MODE FOR CARRYING OUT THE INVENTION

As described above, according to a first aspect, the present invention provides a method for inducing antigen-specific T cells in a patient comprising administering to said patient in need thereof composition (a) which comprises a therapeutically effective amount of an antigen protein or an antigen peptide as an active ingredient and composition (b) which comprises a therapeutically effective amount of a cell wall skeleton integrant of the BCG strain of *Mycobacterium bovis* as an active ingredient, wherein said composition (b) is administered in advance and then said composition (a) is administered. This method is characterized in that it comprises an administration procedure in which the BCG-CWS as an adjuvant is administered in advance, and then, after a certain period, an antigen (an antigen protein or antigen peptide derived from said antigen protein) is administered. The administration procedure of the present invention can drastically improve an anti-tumor effect, antiviral effect, and disease free survival rate, compared to the administration of the antigen alone, or the BCG-CWS alone.

The term "antigen protein or antigen peptide" comprised as an active ingredient in composition (a) refers to an antigen protein and an antigen peptide derived from said antigen protein, and is not specifically limited so long as it is capable of inducing T cells specific, for an antigen peptide. In addition, the term "antigen protein or antigen peptide" also includes within its scope both those capable of inducing antigen-specific T cells directly by forming a complex with an MHC molecule (HLA molecule) on the cell surface of an antigen-presenting cell as well as those capable of inducing antigen-specific T cells indirectly, that is, by being incorporated into the cell and intracellularly degraded to a peptide fragment which in turn binds an MHC molecule to form a complex presented on the cell surface.

Antigen proteins include, for example, antigen proteins derived from viruses, antigen proteins derived from bacteria, or cancer antigen proteins (also referred to as tumor antigen proteins). Several proteins already known as antigen proteins are listed below. Examples of antigen proteins derived from viruses are those derived from HIV, hepatitis C virus, hepatitis B virus, influenza virus, HPV, HTLV, and EBV. Examples of antigen proteins derived from bacteria are those derived from tubercle bacilli. Representative examples of cancer antigen proteins are those listed in Table 1 of Immunity, vol. 10: 281, 1999, or those listed in Tables 1 to 6 of Cancer Immunol. Immunother., vol. 50, 3-15, 2001. More specifically, melanoma antigen proteins include MAGE (Science, 254:1643, 1991), gp 100 (J. Exp. Med., 179:1005, 1994), MART-1 (Proc. Natl. Acad. Sci. USA, 91:3515, 1994), and tyrosinase (J. Exp. Med., 178:489, 1993); and cancer antigen proteins other than those derived from melanoma includes tumor makers such as HER2/neu (J. Exp. Med., 181:2109, 1995), CEA (J. Natl. Cancer. Inst., 87:982, 1995), and PSA (J. Natl. Cancer. Inst., 89:293, 1997), as well as SART-1 derived from squamous cell carcinoma (J. Exp. Med., vol. 187, p. 277-288, 1998; WO 97/46676), cyclophilin B (Proc. Natl. Acad. Sci., U.S.A. 88:1903, 1991), SART-3 (Cancer Res., vol. 59, 4056 (1999)), and WT1 (Immunogenetics, vol. 51, 99, 2000; Blood 95:2198-203, 2000; Blood 95: 286-93, 2000, or human WT1 set forth in the Sequence Listing of the present application as SEQ ID NO: 1). In addition to the above antigen proteins in their full-length forms, partial polypeptides or alterations thereof are also included so long as they are capable of inducing T cells specific for an antigen peptide.

Such antigen proteins may be obtained through the following steps: cloning a cDNA encoding a desired antigen protein, ligating the cDNA into an expression vector, introducing the resulting recombinant expression vector into a host cell, and expressing the antigen protein, according to the references cited above or to standard texts such as Molecular Cloning 2nd Ed., Cold Spring Harbor Laboratory Press (1989). More specifically, for example, a cDNA encoding a desired antigen protein is cloned by hybridization or a PCR method. The cloned cDNA is then incorporated into an appropriate expression vector (e.g. pSV-SPORT1). The resulting recombinant expression vector is introduced into a host cell, and the transformants thus obtained may be cultured in an appropriate medium to express and produce the desired antigen protein. In this context, host cells include, for example, prokaryotes such as *E. coli*, unicellular eukaryotes such as yeasts, and multicellular eukaryotic cells such as those of insects or animals. Methods of gene transfer into host cells include, for example, the calcium phosphate, DEAE-dextran, and electric pulse methods. Polypeptides thus obtained can be isolated and purified using standard biochemical techniques.

The *in vitro* ability of such antigen proteins to induce antigen-specific T cells can be examined, for example, in the case of cancer antigen proteins, by a test as described below. Thus, first, certain cells which express no cancer antigen protein such as COS-7 derived from African green monkey kidney (ATCC CRL1651) or fibroblast VA-13 (RIKEN Cell Bank, The Institute of Physical and Chemical Research) are double transfected with a recombinant expression vector which comprises cDNA encoding a desired cancer antigen protein and a recombinant expression vector which comprises DNA encoding an HLA antigen. Such transfection may be achieved by the Lipofectin method using Lipofectamine reagent (GIBCO BRL). Subsequently, tumor-reactive CTLs restricted by the HLA molecule used are added and allowed to act. The amounts of various cytokines (e.g. IFN-γ) produced by said CTLs in response may be then measured, for example, by an ELISA method, in order to evaluate an activity of the desired cancer antigen protein to induce antigen-specific T cells.

Antigen peptides derived from antigen proteins (hereinafter simply referred to as antigen peptides) include, for example, peptides of about 8 to 20 amino acid residues which are part of said antigen proteins, or altered peptides thereof having functionally equivalent properties, or polytopes in which two or more of said peptides or altered peptide thereof are linked together. In this definition, the range "8 to 20" is based on the common knowledge among those skilled in the art that antigen peptides presented by MHC molecules are usually about 8 to 20 amino acids in length. The term "altered peptide having functionally equivalent properties" means an altered peptide in which one to several amino acid residues in the amino acid sequence of an antigen peptide have been substituted, deleted, and/or added (including addition to the amino acid at the N- or C-terminal end of the peptide) and which is capable of inducing T cells specific for an antigen peptide.

In cancer antigen peptides and virus-derived antigen peptides, certain rules (motifs) in the sequences of antigen peptides bound and presented by an HLA molecule are known for certain HLA types such as HLA-A1, -A0201, -A0204, -A0205, -A0206, -A0207, -A11, -A24, -A31, -A6801, -B7, -B8, -B2705, -B37, -Cw0401, and -Cw0602 (see, e.g., Immunogenetics, 41:178, 1995). For example, regarding a motif for HLA-A24, it is known that the amino acid at position 2 in a peptide consisting of 8 to 11 amino acids is tyrosine, phenylalanine, methionine, or tryptophan, and the amino acid at the C-terminal end is phenylalanine, leucine, isoleucine, tryptophan, or methionine (J. Immunol., 152, p. 3913, 1994; Immunogenetics, 41, p. 178, 1995; J. Immunol., 155, p. 4307, 1994). Likewise, regarding HLA-A2, motifs listed below in Table 1 are known (Immunogenetics, 41, p. 178, 1995; J. Immunol:, 155, p. 4749, 1995).

**Table 1**

| Type of HLA-A2 | Amino acid at position 2 from the N-terminal end | Amino acid at the C-terminal end |
|---|---|---|
| HLA-A0201. | L, M | V, L |
| HLA-A0204 | L | L |
| HLA-A0205 | V, L, I, M | L |
| HLA-A0206 | V, Q | V, L |
| HLA-A0207 | L | L |
| (the peptides are 8 to 11 amino acids in length) | | |

Furthermore, in recent years, peptide sequences expected to be able to bind HLA antigens can be searched on the Internet by using a software of BIMAS at NIH (http://bimas.dcrt.nih.gov/molbio/hla _bind/). Such peptide sequences also can be searched by using BIMAS HLA peptide binding prediction analysis (J. Immunol., 152,163, 1994).

It is therefore easy to select antigen peptide portions involved in these motifs from the amino acid sequences of the cancer antigen proteins or virus-derived antigen proteins as described above. Specific examples of antigen peptides thus selected, in particular, for example, of cancer antigen peptides, are as follows. Examples of cancer antigen peptides derived from WT1 are peptides listed in Table II to Table XLVI of WO 2000/18795, and in particular, peptides having the HLA-A24 and HLA-A2 binding motifs set forth in the Sequence Listing of the present application as SEQ ID NOs: 2 and 3. Examples of cancer antigen peptides derived from SART-1 are peptides listed in the Sequence Listings of WO 97/46676, WO 2000/02907, and WO 2000/06595. Examples of cancer antigen peptides derived from cyclophilin B are peptides listed in the Sequence Listing of WO 99/67288. Examples of cancer antigen peptides derived from SART-3 are peptides listed in the Sequence. Listing of WO 2000/12701. By subjecting the above peptides to an activity measurement described below, one can select antigen peptides having an activity to induce antigen-specific T cells.

In addition, where certain rules (motifs) in the antigen peptide sequences that are bound and presented by an HLA molecule are known as described above, altered peptides having properties functionally equivalent to those of the above antigen peptides may be exemplified by altered peptides in which one or more of amino acids have been substituted on the basis of said motifs. Thus, in the case of a binding motif for HLA-A24, for example, it is known as described above that the amino acid at position 2 in a peptide consisting of 8 to 11 amino acids is tyrosine, phenylalanine, methionine, or tryptophan, and the amino acid at the C-terminal end is phenylalanine, leucine, isoleucine, tryptophan, or methionine (J. Immunol., 152, p. 3913, 1994; Immunogenetics, 41, p. 178, 1995; J. Immunol., 155, p. 4307, 1994). Therefore, altered peptides bound and presented by the HLA-A24 antigen may be exemplified by those in which one or more amino acids at position 2 and at the C-terminal end of an HLA-A24-restricted wild-type peptide have been substituted within the amino acids listed above. Specific examples of such altered peptides, for example, in connection with cancer antigens, are as follows. Examples of altered peptides derived from WT1 are those obtained by modifying the peptides listed in Table II to Table XLVI of WO2000/18795 on the basis of the above motif, and in particular, a peptide having the amino acid sequence set forth in the Sequence Listing of the present application as SEQ ID NO: 4. Likewise, examples of altered peptides derived from, for example, SART-1, cyclophilin B, or SART-3 are those obtained by modifying respective antigen peptides disclosed in the above references on the basis of the motif.

Antigen peptides (including altered peptides) as described above may be prepared according to the methods usually used in peptide chemistry. Examples of such methods are those described in references, for example, "Peptide Synthesis", Interscience, New York, 1966; "The Proteins", Vol. 2, Academic Press Inc., New York, 1976; "Peputido-Gosei", Maruzen, 1975; "Pepuchido-Gosei-no-Kiso-to-Jikken", Maruzen, 1985; and "Iyakuhin-no-Kaihatu, Zoku, Vol. 14, Peputido-Gosei", Hirokawa Shoten, 1991. Alternatively, such peptides may also be prepared according to "Molecular Cloning" cited above by expressing a recombinant peptide from a DNA encoding the antigen peptide and purifying it using routine procedures.

The *in vitro* ability of such antigen peptides to induce antigen-specific T cells can be examined, for example, in the case of cancer antigen peptides, by an assay described, for example, in J. Immunol., 154, p. 2257, 1995. In particular, peripheral blood lymphocytes may be isolated from an HLA antigen-positive human, and stimulated *in vitro* by adding a peptide of interest. If CTLs that specifically recognize the HLA-positive cells pulsed with the peptide are induced, the peptide can be thereby confirmed to have an activity to induce antigen-specific T cells. The presence or absence of CTL induction may be determined, for example, by measuring the amount of IFN-γ produced by CTLs in response to the antigen peptide-presenting cells using an enzyme-linked immunosorbent assay (ELISA). Alternatively, the amount of TNF-α produced by CTLs in response to the antigen peptide-presenting cells may be determined by measuring the survival rate of a TNF-α sensitive cell line (e.g. WEHI164S cells; ATCC Cat. No. CRL-1751).

Determination may also be achieved by a method in which the cytotoxicity of CTLs against antigen peptide-presenting cells labeled with ⁵¹Cr is measured (⁵¹Cr release assay; Int. J. Cancer, 58:317, 1994). Alternatively, for example, COS-7 (ATCC No. CRL1651) or VA-13 (RIKEN Cell Bank, The Institute of Physical and Chemical Research) cells into which an expression plasmid expressing cDNA for HLA has been introduced are pulsed with a peptide of interest. Then, for example, the CTLs prepared as described above may be reacted with the pulsed cells, and the amounts of various cytokines (e.g. IFN-γ or TNF-α) produced by said CTLs may be measured.

The term "polytope" means a recombinant peptide in which two or more antigen peptides have been linked together (see, e.g., Journal of Immunology, 160, p. 1717, 1998), and particularly in the present invention refers to a polypeptide which appropriately combines one, two or more kinds of the above antigen peptides. A polytope is obtained by a procedure in which a recombinant DNA prepared by linking together one, two or more kinds of DNAs encoding the above antigen peptides is inserted into an appropriate expression vector, and the recombinant vector obtained is then expressed in a host cell. Activity of the polytope to induce antigen-specific T cells may be confirmed by subjecting it to the assay for antigen protein as described above.

At least one kind of antigen protein or peptide as described above is selected and used as an active ingredient in the above composition (a). Depending on the purpose, two or more kinds of antigen proteins or peptides may be present. Although a therapeutically effective amount of such antigen proteins or peptides is not specifically limited so long as it is capable of inducing *in vivo* antigen-specific T cells, it is preferably usually 0.0001 mg to 1000 mg, more preferably 0.001 mg to 100 mg, still more preferably 0.01 mg to 10 mg.

The above composition (a) is preferably formulated into a dosage form that achieves desired pharmacological effects. Dosage forms suitable for this purpose include, for example, formulations such as water-in-oil (w/o) emulsions, oil-in-water (o/w) emulsions, and water-in-oil-in-water (w/o/w) emulsions, as well as liposome formulations, microsphere formulations, microcapsule formulations, solid injections and liquid formulations.

Water-in-oil (w/o) emulsion formulations take the form in which an active ingredient is dispersed in an aqueous dispersed phase. Oil-in-water (o/w) emulsion formulations take the form in which an active ingredient is dispersed in an aqueous dispersion medium. Likewise, water-in-oil-in-water (w/o/w) emulsion formulations take the form in which an active ingredient is dispersed in the inner-most aqueous dispersed phase. Preparation of such formulations may be achieved by referring to, for example, JP Kokai H08-985, JP Kokai H09-122476, etc.

Liposome formulations comprise microparticles in the form in which an active ingredient is incorporated in an aqueous phase or within membranes by means of liposomes having a lipid bilayer structure. Examples of principal lipids for preparing liposomes are phosphatidylcholine and sphingomyelin, to which, for example, dicetyl phosphate, phosphatidic acid, or phosphatidylserine is added to provide liposomes with electric charges for stabilization. Exemplary methods for preparing liposomes are ultrasonication, ethanol injection, ether injection, reverse-phase evaporation, and French press extraction methods.

Microsphere formulations comprise microparticles made of a homogeneous polymer matrix in which an active ingredient is dispersed. Examples of components for the matrix are biodegradable polymers such as albumin, gelatin, chitin, chitosan, starch, polylactic acid, and polyalkyl cyanoacrylate. Preparation of microsphere formulations may be carried out according to known methods (Eur. J. Pharm. Biopharm. 50:129-146, 2000; Dev. Biol. Stand. 92:63-78, 1998; Pharm. Biotechnol. 10: 1-43, 1997) and is not specifically limited.

Microcapsule formulations comprise microparticles in the form in which an active ingredient as a core substance is covered with an encapsulating substance. Examples of a coating material used as an encapsulating substance are membrane-forming polymers such as carboxymethylcellulose, cellulose acetate phthalate, ethylcellulose, gelatin, gelatin-acacia, nitrocellulose, polyvinyl alcohol, and hydroxypropylcellulose. Microcapsule formulations may be prepared according to, for example, the coacervation or interfacial polymerization method.

Solid injections are dosage forms in which an active ingredient is included in a base material such as collagen or silicone to solidify the forms. Solid injections may be prepared according to, for example, a method described in a reference (Pharm. Tech. Japan, 7 (1991), p. 402-409).

Liquid formulations are dosage forms in which an active ingredient is mixed with a pharmaceutically acceptable solvent, carrier, or the like. Examples of a pharmaceutically acceptable solvent include water, a glucose solution, and physiological saline. In addition, liquid formulations may comprise a pharmaceutically acceptable auxiliary agent such as a pH regulating agent or buffer, a tonicity adjusting agent, or a swelling agent.

Furthermore, composition (a) may also take the form of a lyophilized formulation corresponding to the above dosage forms. Other agents such as a stabilizing agent (e.g. polysaccharides, amino acids, proteins, urea, or sodium chloride), an excipient (e.g. sugars, amino acids, urea, or sodium chloride), an antioxidant, an antiseptic, an isotonizing agent, or an buffer may also be added if necessary.

Such composition (a) as described above may be used as a pre-formulated product or may be prepared before use for administration to a patient. Thus, the antigen protein or the antigen peptide as an active ingredient of composition (a) as well as an emulsion or other preparation as the dosage form may be used as a pre-formed product in which the constituents have already been mixed together, or may be prepared before use for administration to a patient.

Composition (b), that is a composition comprising as an active ingredient a BCG-CWS is described below.

BCG-CWS that is a CWS of the BCG strain of *Mycobacterium bovis* may be isolated and prepared according to known literatures such as Cancer Res., 33, 2187-2195 (1973), J. Natl. Cancer Inst., 48, 831-835 (1972), J. Bacteriol., 94; 1736-1745 (1967), Gann, 69, 619-626 (1978), J. Bacteriol., 92, 869-879 (1966), and J. Natl. Cancer Inst., 52, 95-101 (1974). In brief, those CWSs may be obtained in a form of insoluble residue by a purification process which comprises crashing the cells with a physical means, removing nucleic acids and proteins from the cell debris, and then delipidating the resultant material.

Said BCG-CWS is preferably formulated into a dosage form that achieves a desired pharmacological effect. In order to attain the purpose, an emulsion is preferable, and an oil-in-water (o/w) emulsion is more preferable. Oils as a constituent component of the oil-in-water emulsion include mineral oils or animal and vegetable oils as described in Immunology, 27, 311-329 (1974). The mineral oil is exemplified by a liquid petrolatum, a bayol (Bayol F), Drakeol-6VR, and the like. The vegetable oil is exemplified by a peanut oil, a sesame oil, AD-65 (a mixture of a peanut oil, Arlacel, and aluminum monostearate), and the like. The animal oil is exemplified by squalane, and a terpenoid derivative such as squalene, and the like. Among them, Drakeol-6VR and squalane are preferred.

BCG-CWS is preferably comprised in the oil-in-water emulsions in a concentration range of 0.1 to 10 mg/ml. Oil is suitably comprised in a concentration range of 0.01 to 30 %w/w, preferably 0.01 to 10% w/w, and more preferably 0.01 to 5.0 %w/w.

Oil-in-water emulsion formulations may comprise a surfactant, a stabilizer, an excipient or the like, if necessary. In this context, a surfactant is not limited to a particular species as long as it may be used in a pharmaceutical formulation. It includes a phospholipid, a nonionic surfactant, and the like. Specific examples include phospholipids such as egg-yolk phosphatidyl amine, egg-yolk lecithin, soybean lecithin, nonionic surfactants such as polyoxyethylene sorbitan fatty acid esters, e.g. Polysorbate 80, and sorbitan fatty acid esters, e.g. Span 40. Each surfactant may be used solely, or in combination with any other several ones, if necessary.

Said stabilizers include a polysaccharide, an amino acid, a protein, urea, a sugar alcohol, and sodium chloride. Specific examples include polysaccharides such as dextran, starch, cellulose, and amino acids such as a neutral amino acid, e.g. alanine, glycine and proline. Proteins include albumin, a gelatin, and a collagen. Sugar alcohols include mannitol and sorbitol. Each stabilizer may be used solely, or in combination with any other several ones, if necessary.

Said excipients include a saccharide, an amino acid, urea, and sodium chloride. Specific examples include saccharides such as a monosaccharide, a disaccharide, and a sugar alcohol. Monosaccharides include glucose, and fructose, and disaccharides include maltose, lactose, and trehalose. Sugar alcohols include mannitol, and sorbitol. Amino acids includes alanine, and glycine. Each excipient may be used solely, or in combination with any other several ones, if necessary.

In addition, an antioxidant, an antiseptic, an isotonizing agent, or an buffer, each of which may be used in pharmaceutical formulations may be comprised, if necessary.

Oil-in-water emulsion formulations that comprise BCG-CWS as described above may also take the form of a lyophilized formulation. A dispersion solvent to be used to re-disperse the lyophilized formulation is a medium for dispersing emulsion particles, and includes injectable water (injectable distilled water), a physiological saline and the like, but is limited to a particular species as long as it can be injected as a dispersion solvent.

Details for the processes of preparation of such an oil-in-water emulsion formulation that comprises BCG-CWS as described above are discussed in WO00/3724. Specifically, the emulsion may be prepared by, for example, adding a BCG-CWS to an oil as described above, adding further an aqueous solution of a surfactant, an excipient, a stabilizer and another additive, and then emulsifying the resultant mixture with a dispersing or emulsifying device such as a Potter-Elvehjem type homogenizer, a homomixer, an ultrasonic homogenizer, Microfluidizer, or the like, followed by lyophilizing the oil-in-water emulsion to give finally a lyophilized formulation.

Such composition that comprises a BCG-CWS as an active ingredient in composition (b) as prepared as shown above may be used as a pre-formulated product, or may be prepared before use for administration to a patient. Therapeutically effective amount of BCG-CWS is not limited to specific one as long as it facilitates the enhancement of the activity to induce antigen-specific T cells, and may be preferably 0.1 to 200 µg, and more preferably 1 to 100 µg per administration.

A method of inducing antigen-specific T cells according to the present invention which comprises administering compositions (a) and (b) as described above is characterized in that composition (b) is administered in advance and then composition (a) is administered. In particular, composition (a) is preferably administered 6 or more hours after the administration of composition (b), and more preferably composition (a) is administered 12 or more hours after the administration of composition (b). Further preferably, composition (a) is administered about 12 to 48 hours after the administration of composition (b), and still more preferably, composition (a) is administered about 24 to 48 hours after the administration of composition (b). The most preferable timing of administration is such that composition (a) is administered about 24 hours (ca. 1 day; 20 to 28 hours) after the administration of composition (b).

In this connection, administration may be carried out in any manner so long as the timing of administration is such that composition (b) is administered in advance and then composition (a) is administered, as described above, and examples of the administration procedure include:
1) composition (b) is administered one or more times, and after a certain period as described above, composition (a) is administered; or
2) composition (b) is administered one or more times, and after a certain period as described above, compositions (b) and (a) are simultaneously administered.

In this regard, when composition (b) is administered two or more times, the number of administrations may be in particular 2 to 10, and preferably 2 to 5.

Taking the above administrations of (a) and (b) as one administration cycle, such an administration cycle may be repeated two or more times in order to further improve the effect on induction of T cells. Thus, the administration cycle may be repeated two or more times as appropriate depending, for example, on the disease to be treated, the symptoms, age, and weight of the patient. The interval between the repeated administration cycles may also be determined as appropriate in the range from about one week to about one year. depending, for example, on the symptoms of the patient.

The route of administration for compositions (a) and (b) used in a method of inducing antigen-specific T cells according to the present invention may be, for example, intradermal administration, subcutaneous administration, continuous subcutaneous administration, intravenous injection, intraarterial injection, intramuscular injection, local infusion, or intraperitoneal administration. It is also possible to continuously and slowly administer using, for example, an osmotic pump, or to prepare a sustained-release formulation (e.g. a mini-pellet formulation) and implant it. Preferred is intradermal or subcutaneous administration. It is particularly preferred to intradermally administer both of compositions (a) and (b). In that case, it is preferred to intradermally administer compositions (a) and (b) at the same site.

Exemplary combinations of the active ingredients of the above compositions (a) and (b) in a method of inducing antigen-specific T cells according to the present invention may be combinations of an antigen protein with a BCG-CWS, or may also be combinations of an antigen peptide with a BCG-CWS. Among these combinations, when the antigen is a cancer antigen, specific examples include a combination of WT1 protein (SEQ ID NO: 1) with a BCG-CWS and a combination of a cancer antigen peptide derived from WT1 with a BCG-CWS. In this context, the cancer antigen peptide derived from WT1 may be those peptides found in the amino acid sequence of human WT1 set forth in SEQ ID NO: 1 which have a motif structure as described above that is bound and presented by an HLA antigen, as well as altered peptides thereof. Particular examples are the peptides listed in Table II to Table XLVI of WO 2000/18795 and altered peptides based on the motifs, and more preferable, Arg Met Phe Pro Asn Ala Pro Tyr Leu (SEQ ID NO: 2) and Cys Met Thr Trp Asn Gln Met Asn Leu (SEQ ID NO: 3) which have a binding motif for HLA-A2 and HLA-A24, or altered peptides thereof based on the HLA-A24 or HLA-A2 binding motif. A specific example of such altered peptides is Cys Tyr Thr Trp Asn Gln Met Asn Leu (SEQ ID NO: 4) in which Met at position 2 of the peptide set forth in SEQ ID NO: 3 has been replace by Tyr, an amino acid consistent with the motif.

A method of inducing antigen-specific T cells according to the present invention as described above may be examined for its ability to induce antigen-specific T cells as follows.

Composition (b) according to the present invention is injected intradermally into a laboratory animal, and after 24 hours, composition (a) is injected intradermally. Taking these administrations as one course, vaccination is conducted once or several times at intervals of one to two weeks. One week after the last administration, the spleen is removed, and lymphocytes are prepared from the spleen. Splenocytes from unprimed mice are also prepared in parallel, and pulsed with an antigen peptide for several hours followed by X-irradiation at a dose of about 2000 to 5000 rad to use as antigen-presenting cells. Lymphocytes from immunized mice are restimulated with the antigen peptide in a culture system by adding thereto the antigen-presenting cells. If necessary, similar stimulation is conducted several times at a frequency of once a week. One week after the last stimulation, lymphocytes are recovered, and may be examined for their ability to induce antigen-specific T cells using target cells such as cells pulsed with the antigen peptide or cells positive for the antigen, for example, by determining the amounts of various cytokines (e.g. IFN-γ) produced in response by antigen peptide-specific T cells induced among lymphocytes, or by measuring the cytotoxicity of antigen peptide-specific T cells against target cells labeled with ⁵¹Cr according to the ⁵¹Cr release assay (J. Immunol., 139:2888, 1987). For human, peripheral blood mononuclear cells (PBMCs) isolated from peripheral blood, for example, by the Ficoll method may be used instead of splenic lymphocytes of laboratory animals in order to examine, in a similar manner, the ability to induce antigen peptide-specific T cells.

It is also possible to examine the ability to induce cancer antigen-specific T cells by the procedures described below in the Examples. Briefly, cDNA encoding a cancer antigen protein of interest is introduced into a tumor cell to prepare tumor cells highly expressing the cancer antigen protein of interest. The tumor cells are administered intraperitoneally, and vaccination is started on the next day. Vaccination is achieved by four courses of administration conducted at intervals of one week; one course of administration consisting of first intradermally injecting composition (b) as described above, and after 24 hours, intradermally injecting composition (a). Then, anti-tumor effects based on the ability to induce antigen-specific T cells may be examined by measuring a tumorigenic rate, a survival rate, or a disease-free survival rate using a routine method. Alternatively, an administration procedure similar to that described above may be conducted on a tumor patient instead of a laboratory animal to examine the ability to induce antigen peptide-specific T cells.

When applied to an antigen-positive patient, a method of inducing antigen-specific T cells according to the present invention can cause an antigen peptide to be presented at a high density with an HLA antigen on antigen-presenting cells and can thereby induce proliferation of T cells specific for the presented HLA antigen-peptide complex, leading to killing of target cells (cells positive for the antigen peptide) or activation of immunity by production of various cytokines. A method of inducing antigen-specific T cells according to the present invention, where the antigen is a cancer antigen, is used for treatment or prevention of a cancer. In particular; it is used for treatment or prevention of, for example, lung, ovarian, and prostatic cancer as well as leukemia. Likewise, the method is used for treatment or prevention of viral infections, where the antigen is a virus-derived antigen.

In treatment or prevention of a cancer, a method of inducing antigen-specific T cells according to the present invention can induce and enhance specific cellular immunity against cancer cells, and thereby treat a cancer or prevent proliferation and metastasis of a cancer. In addition, a method of inducing antigen-specific T cells according to the present invention may be used in combination with a conventional chemotherapy or radiotherapy to enhance the therapeutic effects. In treatment or prevention of a viral infection, a method of inducing antigen-specific T cells according to the present invention can induce and enhance specific cellular immunity against virus-infected cells, and can thereby treat or prevent a virus infection.

Although the timing of starting a method of inducing antigen-specific T cells according to the present invention is not specifically limited, the method may be preferably carried out, for example, after a patient with leukemia has attained complete remission (CR), or during situations in which the number of tumor cells has been reduced by a solid cancer surgery, that is, the patient has achieved a state of minimal residual disease (MRD).

In connection with the above embodiments, the present invention provides a method of treatment and/or prevention of a cancer in a patient comprising a method for inducing antigen-specific T cells in the patient according to the present invention.

In another embodiment, the present invention relates to a pharmaceutical composition for enhancing an activity of an antigen protein or an antigen peptide to induce antigen-specific T cells, which comprises a BCG-CWS as an active ingredient, and which is administered before the administration of the antigen protein or the antigen peptide; and
to a pharmaceutical composition for enhancing an anticancer activity based on the immunopotentiating action of a BCG-CWS, which comprises an antigen protein or an antigen peptide as an active ingredient and which is administered after the administration of said non-specific immunopotentiator, wherein the anticancer activity is enhanced by the activity to induce antigen-specific T cells. The compositions comprising a BCG-CWS as described above may be prepared and used according to the above descriptions for composition (b), while the compositions comprising an antigen protein or an antigen peptide as described above may be prepared and used according to the above descriptions for composition (a).

In connection with the above embodiments, the present invention provides a use of a BCG-CWS for preparing a medicament which is administered before the administration of an antigen protein or an antigen peptide and which enhances an activity of the antigen protein or the antigen peptide to induce antigen-specific T cells, as well as a use of an antigen protein or an antigen peptide for preparing a medicament which is administered after the administration of a BCG-CWS and which enhances an anticancer activity based on the immunopotentiating action of said BCG-CWS, wherein the anticancer activity is enhanced by the activity of the cancer antigen protein or the cancer antigen peptide to induce antigen-specific T cells.

In particular embodiments, the present invention also provides a pharmaceutical composition for enhancing an activity of WT1 protein set forth in SEQ ID NO: 1 or a cancer antigen peptide derived from said WT1 protein to induce antigen-specific T cells, which comprises a BCG-CWS as an active ingredient, as well as a pharmaceutical composition for enhancing an anticancer activity based on the immunopotentiating action of a BCG-CWS, which comprises as an active ingredient WT1 protein set forth in SEQ ID NO: 1 or a cancer antigen peptide derived from said WT1 protein wherein the anticancer activity is enhanced by the activity of the protein or the peptide to induce antigen-specific T cells. The present embodiments encompass a pharmaceutical composition for enhancing an activity of WT1 protein set forth in SEQ ID NO: 1 or a cancer antigen peptide derived from said WT1 protein to induce antigen-specific T cells, which comprises WT1 protein set forth in SEQ ID NO: 1 or a cancer antigen peptide derived from said WT1 protein along with a BCG-CWS, and a pharmaceutical composition for enhancing an anticancer activity based on the immunopotentiating action of a BCG-CWS, which comprises WT1 protein set forth in SEQ ID NO: 1 or a cancer antigen peptide derived from said WT1 protein along with a BCG-CWS.

As shown in Examples hereinafter, when used in combination with BCG-CWS for the first time, WT1 has been demonstrated to have the effects on the treatment of tumors and the effects on the improvement in disease free survival rate. The effects were much better than those on the approaches wherein an antigen or a BCG-CWS is solely administered. It is therefore expected that a therapeutic and/or prophylactic agent for cancer which involves a combination of WT1 and BCG-CWS, or a therapeutic and/or prophylactic agent for cancer which involves a combination of a cancer antigen peptide derived from said WT1 would exert clinical effects as an excellent anti-tumor-specific immunotherapeutic agent. When WT1 is used in combination with BCG-CWS, any one of WT1 and BCG-CWS may be administered first, or they may be mixed together before the administration.

In this context, cancer antigen peptides derived from WT1 may be those peptides found in the amino acid sequence of human WT1 set forth in SEQ ID NO: 1 which have a motif structure as described above that is bound and presented by an HLA antigen, as well as altered peptides thereof. Particular examples are the peptides listed in Table II to Table XLVI of WO 2000/ 18795 and altered peptides based on the motifs, and more preferable, Arg Met Phe Pro Asn Ala Pro Tyr Leu (SEQ ID NO: 2) and Cys Met Thr Trp Asn Gln Met Asn Leu (SEQ ID NO: 3) which have a binding motif for HLA-A2 and HLA-A24, or altered peptides thereof based on the HLA-A24 or HLA-A2 binding motif. A specific example of such altered peptides is Cys Tyr Thr Trp Asn Gln Met Asn Leu (SEQ ID NO: 4) in which Met at position 2 of the peptide set forth in SEQ ID NO: 3 has been replace by Tyr, an amino acid consistent with the motif.

Regarding the therapeutic and/or prophylactic agent for cancer involving a combination of WT1 and the bacterium-derived component as described above, the administration methods, the doses, the dosage forms and the like are similar to those described above for the method of inducing antigen-specific T cells.

In connection with these embodiments, the present invention relates to:
a method for enhancing, in a patient, an activity of WT1 protein set forth in SEQ ID NO: 1 or a cancer antigen peptide derived from said WT1 protein to induce antigen-specific T cells, which comprises administering to said patient a BCG-CWS in an amount effective to enhance the activity to induce antigen-specific T cells;
a method for enhancing, in a patient, an anticancer activity based on the immunopotentiating action of a BCG-CWS, which comprises administering to said patient WT1 protein set forth in SEQ ID NO: 1 or a cancer antigen peptide derived from said WT1 protein in an amount effective to enhance the anticancer activity, wherein the anticancer activity is enhanced by the activity of the protein or the cancer antigen peptide to induce antigen-specific T cells; preferably the method wherein the cancer antigen peptide is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4); and the method as described above for treatment and/or prevention of a cancer.

In connection with those embodiments, the invention relates to a use of a BCG-CWS for preparing a medicament which enhances an activity of WT1 protein set forth in SEQ ID NO: 1 or a cancer antigen peptide derived from said WT1 protein to induce antigen-specific T cells; and a use of WT1 protein set forth in SEQ ID NO: 1 or a cancer antigen peptide derived from said WT1 protein for preparing a medicament which enhance an anticancer activity based on the immunopotentiating action of a BCG-CWS, wherein the anticancer activity is enhanced by the activity of the protein or the cancer antigen peptide to induce antigen-specific T cells; preferably the use wherein the cancer antigen peptide is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4); and the use as described above for preparing a medicament for treatment and/or prevention of a cancer.

### EXAMPLES

The present invention is illustrated below by reference to the following Examples. The present invention is not, however, limited to such Examples.

### Example 1

### Anti-tumor effects of a combination of a WT1 peptide and BCG-CWS

### 1. Materials and Methods

### 1) Cells

C1498, a leukemia cell line, which did not express WT1 derived from a C57BL/6 mouse was purchased from ATCC (Rockville, MD). C1498 (WT1-C1498) cells which expressed mouse WT1 were prepared by transfecting the C1498 cells with the cDNA of mouse WT1 (WO 00/06602) in the usual manner.

### 2) Peptide

A 9-mer peptide which represents a cancer antigen peptide derived from WT1 (sequence: Arg Met Phe Pro Asn Ala Pro Tyr Leu; SEQ ID NO: 2) was synthesized using Fmoc chemistry on an ABI 430A peptide synthesizer (Applied Biosystems Inc., Foster), and then purified by reverse phase chromatography using a C18 Microbondasphere (Waters Japan, Osaka) column. The synthesized peptide was confirmed on an API IIIE triple quadrupole mass spectrometer (Sciex, Toronto, Canada), and the concentration was determined by MicroBCA assay (Pierce, Rockford, IL) using BSA as a standard.

### 3) Preparation of BCG-CWS

BCG-CWS was used as a non-specific immunopotentiator. BCG-CWS was formulated into a form of oil-in-water emulsion according to the previously report (Cancer Res 24: 121-141, 1979). In brief, 9.6 µl of squalane was added to 2 mg of BCG-CWS, and the mixture was homogenized (1200 rpm x 1 minute). To the homogenate was added 1 ml of 0.2% Tween80 PBS, and the mixture was again homogenized (3000 rpm x 8 minutes) to give an emulsion. Recovery rate of BCG-CWS in the emulsion was determined on the basis of concentration of the galactose that was its sugar chain by phenol-sulfuric acid method, and the emulsion having a recovery rate of 30% or more was collected. In the present experiment, the emulsions having a recovery rate between 30% and 40% were used.

### 4) Mice

Male C57BL/6 mice, 6 to 8 weeks old (H-2D^{b}, H-2K^{b}), were purchased from CLEA Japan, Inc.

### 5) Schedule for tumor cell transplantation and vaccination

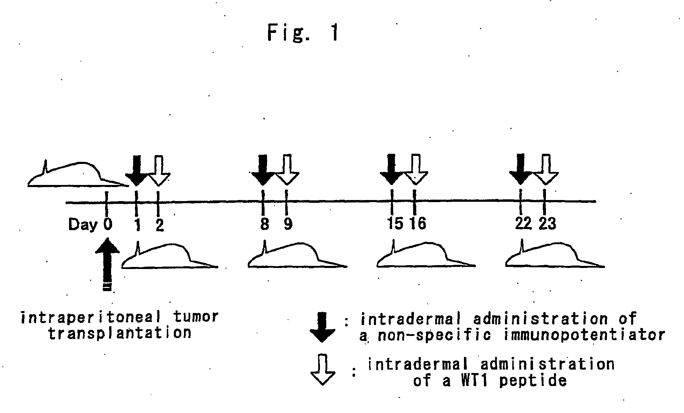

Into mice, 5x10⁵ WT1-C1498 cells were injected intraperitoneally, and vaccination was started on the next day. Into flanks of the mice, 100 µg of BCG-CWS was injected intradermally, and after 24 hours, the WT1 peptide was injected intradermally at the same site. Taking these administrations as one course; four courses were conducted in total at intervals of one week (Fig. 1).

### 6) Colony assay

Colony assay was conduced in the usual manner. In brief, myeloid cells from the mice were seeded on α-MEM containing 20% fetal calf serum, 1% bovine serum albumin Fr5, 1.5% methyl cellulose, and various cytokines (100 ng/ml SCF, 10 ng/ml G-CSF) at 3 x 10⁴ per dish (35mm), and incubated for 14 days, after which colonies having 50 cells or more were counted.

### 7) Detection of WT1-specific CTL activity

The spleens were removed from the three mice in which the coadministration of WT1 peptide and BCG-CWS prohibited any tumor from establishing, and the splenocytes were prepared. Similarly, the splenocytes were also prepared from a control, the untreated mice that had not been vaccinated. WT1-C1498 cells and C1498 cells were labeled with ⁵¹Cr. CTL activity was determined using the splenocytes as effector cells and using the labeled cells as target cells by the ^{SI}Cr release assay (J. Immunol., 159: 4753, 1997):

### 2. Results

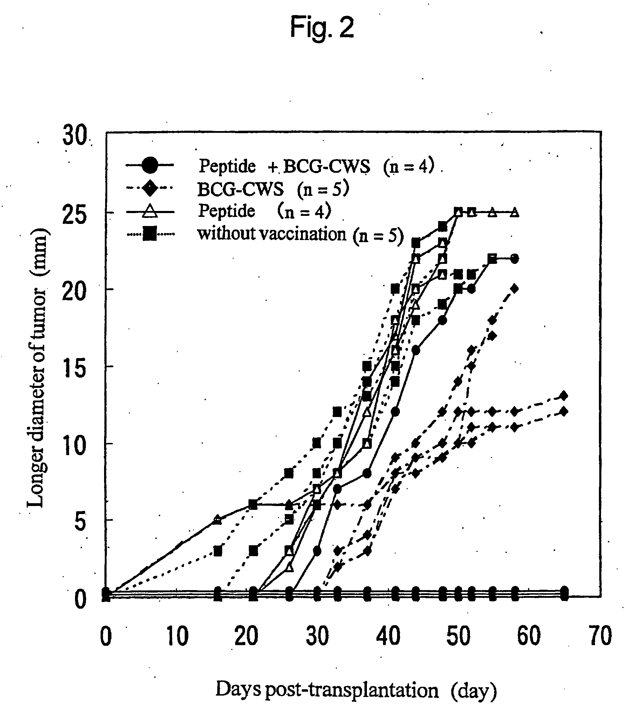

According to the schedule shown in Fig. 1, tumor cell transplantation and vaccine administration were conducted, and the subsequent formation of tumor masses and the survival rate were observed. Fig. 2 shows the tumor diameters in the mice that were transplanted with 5 x 10⁵ cells per animal as observed during the period from the transplantation to Day 65 thereafter. Disappearance of the value curve before Day 65 means that the animals were dead before Day 65. In the group without any vaccination, the tumor masses were palpable at the early stage, and rapidly developed. Similar findings were observed in the group receiving the peptide alone. In the group receiving BCG-CWS alone, the formation of tumor masses was observed to be suppressed, and the rate of tumor mass formation was observed slower compared to those observed in the groups as mentioned above. In the group received the WT1 peptide and BCG-CWS, the formation of tumor masses was further suppressed, and 3 of the 4 mice exhibited no palpable tumor masses even 65 days after the tumor cell transplantation.

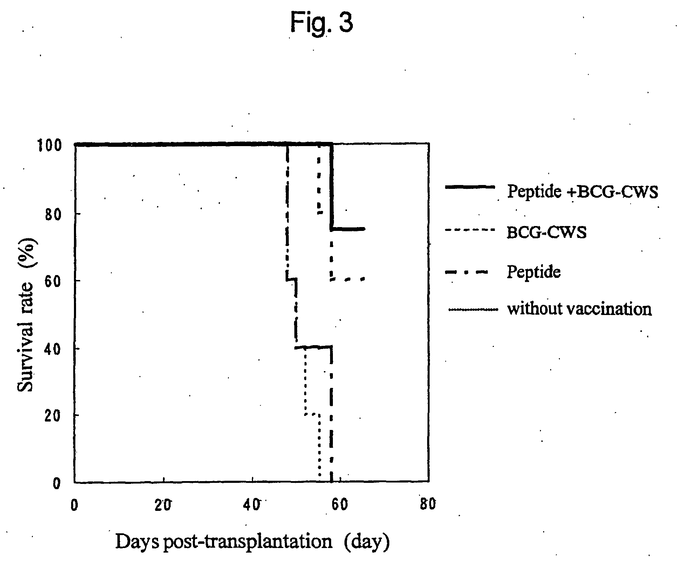

Fig. 3 shows the curve representing survival rate up to Day 65 from the tumor cell transplantation. All of the mice receiving no vaccination were dead on Day 55, and all of the mice receiving the peptide alone were also dead on Day 58. Contrary to these groups, the mice receiving BCG-CWS alone, and the mice receiving the WT1 peptide and BCG-CWS exhibited survival rates of 60% and 75%, respectively.

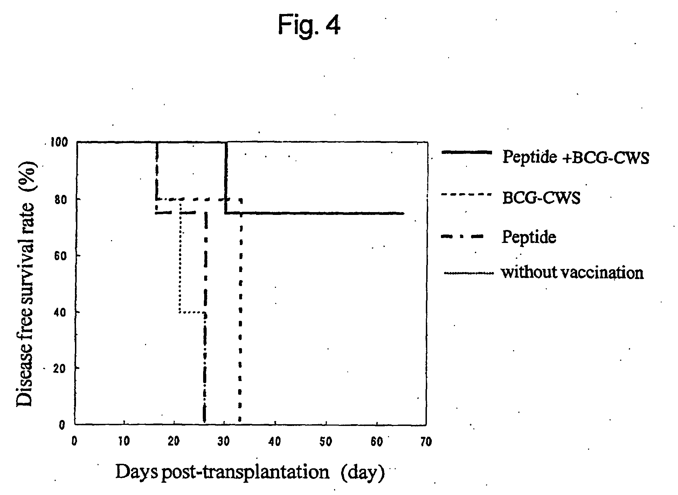

Fig. 4 shows the curve representing disease free survival rate up to Day 65 from the tumor cell transplantation. The group of the mice receiving the peptide alone exhibited a disease free survival rate of 0 % on Day 33, whereas the group of the mice receiving the WT1 peptide and BCG-CWS exhibited a disease free survival rates of 75 % even on Day 65, which was much higher.

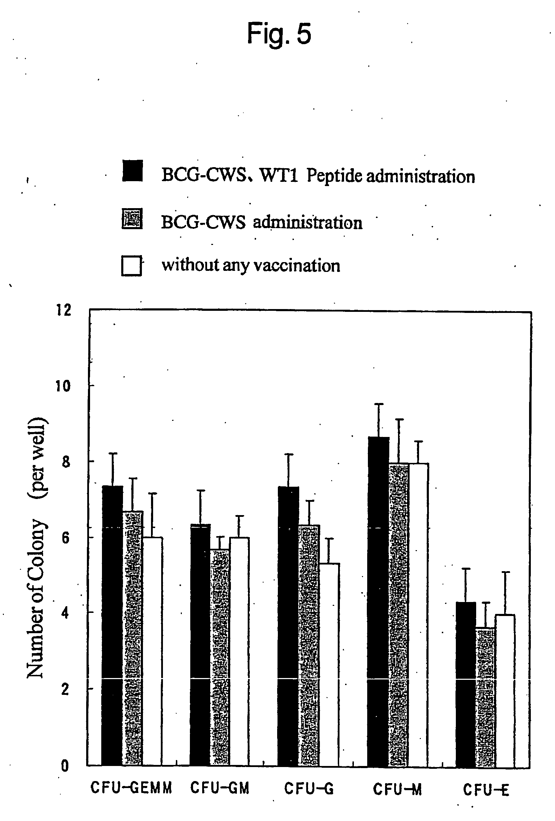

Fig. 5 shows the results of colony assay of the myeloid cells removed on Day 65 from the mice transplanted with tumor cells and vaccinated according to the schedule of Fig. 1. It was shown that the mice vaccinated with WT1 peptide + BCG-CWS and the mice vaccinated with BCG-CWS alone were not significantly different from the mice with neither transplanted tumor cells nor vaccination in terms of the colony numbers of CFU-GEMM, CFU-GM, CFU-G, CFU-M, and CFU-E. These results show that CTLs that attack the tumor cells expressing highly WT1 did not affect the normal cells expressing WT1 (hematopoietic cells in this case).

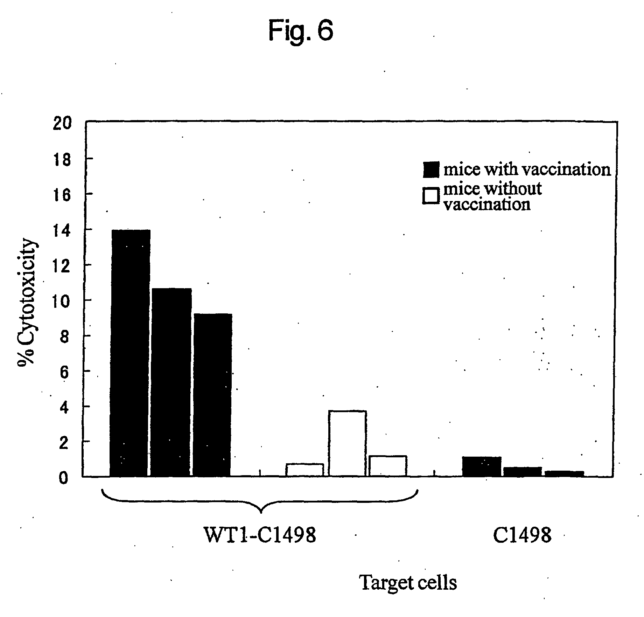

Fig. 6 shows the results of the experiment examining whether or not WT1-specifc CTLs were induced. The splenocytes from all of the 3 mice that were coadministered with the WT1 peptide and BCG-CWS and that any tumor was not established, killed the WT1-C1498 cells, but exhibited no cytotoxic activity on C1498 cells that did not express WT1. The splenocytes from the control, all of the 3 mice without any vaccination exhibited no cytotoxic activity on WT1-C1498 cells. These show that administration of WT1 in combination with BCG-CWS surely induce WT1-specific CTLs.

### INDUSTRIAL APPLICABILITY

The method of the present invention enables efficient induction of antigen-specific T cells. The method of inducing antigen-specific T cells and related pharmaceutical compositions according to the present invention can efficiently, simply and conveniently induce antigen-specific T cells, and are thus useful as anticancer or antiviral agents.

## Claims

1. A method for inducing antigen-specific T cells in a patient comprising administering to said patient in need thereof composition (a) which comprises a therapeutically effective amount of an antigen protein or an antigen peptide as an active ingredient, and composition (b) which comprises a therapeutically effective amount of a cell wall skeleton integrant of the BCG strain of *Mycobacterium bovis* as an active ingredient, wherein composition (b) is administered in advance and then composition (a) is administered.

2. A method according to claim 1, wherein composition (a) is administered about 24 hours after the administration of composition (b).

3. A method according to claim 1 or 2, wherein compositions (a) and (b) are both administered intradermally.

4. A method according to claim 3, wherein compositions (a) and (b) are both administered intradermally at the same site.

5. A method according to any one of claims 1 to 4, wherein the administration cycle involving compositions (a) and (b) is repeated two or more times.

6. A method according to any one of claims 1 to 5, wherein composition (a) comprises a cancer antigen protein or a cancer antigen peptide as an active ingredient.

7. A method according to claim 6, wherein the cancer antigen protein or the cancer antigen peptide is WT1 protein set forth in SEQ ID NO: 1 or a cancer antigen peptide derived from said WT1 protein.

8. A method according to claim 7, wherein the cancer antigen peptide derived from WT1 protein is selected from the group consisting of Arg Met Phe Pro Asn Ala Pro Tyr Leu (SEQ ID NO: 2), Cys Met Thr Trp Asn GIn Met Asn Leu (SEQ ID NO: 3), and Cys Tyr Thr Trp Asn Gln Met Asn Leu (SEQ ID NO: 4).

9. A method of treatment and/or prevention of a cancer in a patient which comprises a method according to any one of claims 1 to 8.

10. A pharmaceutical composition for enhancing an activity of an antigen protein or an antigen peptide to induce antigen-specific T cells, which comprises a cell wall skeleton integrant of the BCG strain of *Mycobacterium bovis* as an active ingredient, and which is administered before the administration of the antigen protein or the antigen peptide.

11. A pharmaceutical composition for enhancing an anticancer activity based on the immunopotentiating action of a cell wall skeleton integrant of the BCG strain of *Mycobacterium bovis,* which comprises an antigen protein or an antigen peptide as an active ingredient, of which the activity to induce antigen-specific T cells facilitates the enhancement of the anticancer activity, said composition being administered after the administration_of the cell wall skeleton integrant of the BCG strain of *Mycobacterium bovis.*

12. A pharmaceutical composition according to claim 10 or 11, wherein the antigen protein or the antigen peptide is a cancer antigen protein or a cancer antigen peptide.

13. A pharmaceutical composition according to claim 12, wherein the cancer antigen protein or the cancer antigen peptide is WT1 protein set forth in SEQ ID NO: 1 or a cancer antigen peptide derived from said WT1 protein.

14. A pharmaceutical composition according to claim 13, wherein the cancer antigen peptide derived from WT 1 is selected from the group consisting of Arg Met Phe Pro Asn Ala Pro Tyr Leu (SEQ ID NO: 2), Cys Met Thr Trp Asn GIn Met Asn Leu (SEQ ID NO: 3), and Cys Tyr Thr Trp Asn Gln Met Asn Leu (SEQ ID. NO: 4).

15. A pharmaceutical composition according to any one of claims 10 to 14 for treatment and/or prevention of a cancer.

16. A use of a cell wall skeleton integrant of the BCG strain of *Mycobacterium bovis* for preparing a medicament which is administered before the administration of an antigen protein or an antigen peptide and which enhances an activity of the antigen protein or the antigen peptide to induce antigen-specific T cells.

17. A use of an antigen protein or an antigen peptide for preparing a medicament which is administered after the administration of a cell wall skeleton integrant of the BCG strain of *Mycobacterium bovis* and which enhances an anticancer activity based on the immunopotentiating action of said a cell wall skeleton integrant of the BCG strain of *Mycobacterium bovis,* wherein the anticancer activity is enhanced by the activity of the cancer antigen protein or the cancer antigen peptide to induce antigen-specific T cells.

18. A pharmaceutical composition for enhancing an activity of WT1 protein set forth in SEQ ID NO: 1 or a cancer antigen peptide derived from said WT1 protein to induce antigen-specific T cells, which comprises a cell wall skeleton integrant of the BCG strain of *Mycobacteriurn bovis* as an active ingredient.

19. A pharmaceutical composition for enhancing an anticancer activity based on the immunopotentiating action of a cell wall skeleton integrant of the BCG strain of *Mycobacterium bovis,* which comprises as an active ingredient WT1 protein set forth in SEQ ID NO: 1 or a cancer antigen peptide derived from said WT1 protein, of which the activity to induce antigen-specific T cells facilitates the enhancement of the anticancer activity.

20. A pharmaceutical composition according to claim 18 or 19, wherein the cancer antigen peptide derived from WT1 is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4).

21. A pharmaceutical composition according to any one of claims 18 to 20 for treatment and/or prevention of a cancer.

22. A method for enhancing, in a patient, an activity of WT1 protein set forth in SEQ ID NO: 1 or a cancer antigen peptide derived from said WT1 protein to induce antigen-specific T cells, which comprises administering to the patient a cell wall skeleton integrant of the BCG strain of *Mycobacterium bovis* in an amount effective to enhance the activity to induce the antigen-specific T cells.

23. A method for enhancing, in a patient, an anticancer activity based on the immunopotentiating action of a cell wall skeleton integrant of the BCG strain of *Mycobacterium bovis,* which comprises administering to the patient WT1 protein set forth in SEQ ID NO: 1 or a cancer antigen peptide derived from said WT1 protein in an amount effective to enhance the anticancer activity, wherein the anticancer activity is enhanced by the activity of the cancer antigen protein or the cancer antigen peptide to induce antigen-specific T cells.

24. A method according to claim 22 or 23, wherein the cancer antigen peptide derived from WT1 protein is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4).

25. A method according to any one of claims 22 to 24 for treatment and/or prevention of a cancer.

26. A use of a cell wall skeleton integrant of the BCG strain of *Mycobacterium bovis* for preparing a medicament which enhances an activity of WT1 protein set forth in SEQ ID NO: 1 or a cancer antigen peptide derived from said WT1 protein to induce antigen-specific T cells:

27. A use of WT1 protein set forth in SEQ ID NO: 1 or a cancer antigen peptide derived from WT1 protein for preparing a medicament which enhances an anticancer activity based on the immunopotentiating action of a cell wall skeleton integrant of the BCG strain of *Mycobacterium bovis,* wherein the anticancer activity is enhanced by the activity of the cancer antigen protein or the cancer antigen peptide to induce antigen-specific T cells.

28. A use according to claim 26 or 27, wherein the cancer antigen peptide derived from WT1 protein is selected from the group consisting of Arg Met Phe Pro Asn Ala Pro Tyr Leu (SEQ ID NO: 2), Cys Met Thr Trp Asn Gln Met Asn Leu (SEQ ID NO: 3), and Cys Tyr Thr Trp Asn Gln Met Asn Leu (SEQ ID NO: 4).

29. A use according to any one of claims 26 to 28 for preparing a medicament for treatment and/or prevention of a cancer.


SEQUENCE LISTING

<110> Haruo Sugiyama and Ichiro Azuma

<120> Method for Inducing Antigen-Specific T Cells

<130> K1553 EP S3

<150> JP 2001-301224
<151> 2001-09-28

<160> 4

<170> PatentIn Ver. 2.1

<210> 1
<211> 449
<212> PRT
<213> Homo sapiens

<400> 1
Met Gly Ser Asp Val Arg Asp Leu Asn Ala Leu Leu Pro Ala Val Pro
  1               5                  10                  15

Ser Leu Gly Gly Gly Gly Gly Cys Ala Leu Pro Val Ser Gly Ala Ala
             20                  25                  30

Gln Trp Ala Pro Val Leu Asp Phe Ala Pro Pro Gly Ala Ser Ala Tyr
         35                  40                  45

Gly Ser Leu Gly Gly Pro Ala Pro Pro Pro Ala Pro Pro Pro Pro Pro
     50                  55                  60

Pro Pro Pro Pro His Ser Phe Ile Lys Gln Glu Pro Ser Trp Gly Gly
 65                  70                  75                  80

Ala Glu Pro His Glu Glu Gln Cys Leu Ser Ala Phe Thr Val His Phe
                 85                  90                  95

Ser Gly Gln Phe Thr Gly Thr Ala Gly Ala Cys Arg Tyr Gly Pro Phe
            100                 105                 110

Gly Pro Pro Pro Pro Ser Gln Ala Ser Ser Gly Gln Ala Arg Met Phe
        115                 120                 125

Pro Asn Ala Pro Tyr Leu Pro Ser Cys Leu Glu Ser Gln Pro Ala Ile
    130                 135                 140

Arg Asn Gln Gly Tyr Ser Thr Val Thr Phe Asp Gly Thr Pro Ser Tyr
145                 150                 155                 160

Gly His Thr Pro Ser His His Ala Ala Gln Phe Pro Asn His Ser Phe
                165                 170                 175

Lys His Glu Asp Pro Met Gly Gln Gln Gly Ser Leu Gly Glu Gln Gln
            180                 185                 190

Tyr Ser Val Pro Pro Pro Val Tyr Gly Cys His Thr Pro Thr Asp Ser
        195                 200                 205

Cys Thr Gly Ser Gln Ala Leu Leu Leu Arg Thr Pro Tyr Ser Ser Asp
    210                 215                 220

Asn Leu Tyr Gln Met Thr Ser Gln Leu Glu Cys Met Thr Trp Asn Gln
225                 230                 235                 240

Met Asn Leu Gly Ala Thr Leu Lys Gly Val Ala Ala Gly Ser Ser Ser
                245                 250                 255

Ser Val Lys Trp Thr Glu Gly Gln Ser Asn His Ser Thr Gly Tyr Glu
            260                 265                 270

Ser Asp Asn His Thr Thr Pro Ile Leu Cys Gly Ala Gln Tyr Arg Ile
        275                 280                 285

His Thr His Gly Val Phe Arg Gly Ile Gln Asp Val Arg Arg Val Pro
    290                 295                 300

Gly Val Ala Pro Thr Leu Val Arg Ser Ala Ser Glu Thr Ser Glu Lys
305                 310                 315                 320

Arg Pro Phe Met Cys Ala Tyr Pro Gly Cys Asn Lys Arg Tyr Phe Lys
                325                 330                 335

Leu Ser His Leu Gln Met His Ser Arg Lys His Thr Gly Glu Lys Pro
            340                 345                 350

Tyr Gln Cys Asp Phe Lys Asp Cys Glu Arg Arg Phe Ser Arg Ser Asp
        355                 360                 365

Gln Leu Lys Arg His Gln Arg Arg His Thr Gly Val Lys Pro Phe Gln
    370                 375                 380

Cys Lys Thr Cys Gln Arg Lys Phe Ser Arg Ser Asp His Leu Lys Thr
385                 390                 395                 400

His Thr Arg Thr His Thr Gly Lys Thr Ser Glu Lys Pro Phe Ser Cys
                405                 410                 415

Arg Trp Pro Ser Cys Gln Lys Lys Phe Ala Arg Ser Asp Glu Leu Val
            420                 425                 430

Arg His His Asn Met His Gln Arg Asn Met Thr Lys Leu Gln Leu Ala
        435                 440                 445

Leu


<210> 2
<211> 9
<212> PRT
<213> Artificial Sequence

<220>
<223> Synthetic Peptide

<400> 2
Arg Met Phe Pro Asn Ala Pro Tyr Leu

<210> 3
<211> 9
<212> PRT
<213> Artificial Sequence

<220>
<223> Synthetic Peptide

<400> 3
Cys Met Thr Trp Asn Gln Met Asn Leu


<210> 4
<211> 9
<212> PRT
<213> Artificial Sequence

<220>
<223> Synthetic Peptide

<400> 4
Cys Tyr Thr Trp Asn Gln Met Asn Leu

3/3

1/3